# EUROPÄISCHE PATENTANMELDUNG

(11) **EP 4 825 957 A2**
(43) Veröffentlichungstag der Anmeldung: **21.10.2026**
(21) Anmeldenummer: 26151882.3
(22) Anmeldetag: 14.01.2026
(51) Int. Cl.: C12M 1/34, C12M 1/36

(54) **VERFAHREN ZUM ERMITTELN EINER ZELLKONZENTRATION EINER DOSIERFLÜSSIGKEIT, DOSIERVORRICHTUNG UND BIOREAKTOR**

(30) Priorität: 31.01.2025 US 202519042780
(71) Anmelder: Festo SE & Co. KG, 73734 Esslingen (DE)
(72) Erfinder: Hahn, Thomas, Sudbury, 01776 (US); Gupta, Sidhaant, Waltham, 02435 (US); Malaviya, Neha, Worcester, 01609 (US)
(74) Vertreter: Patentanwälte Magenbauer & Kollegen Partnerschaft mbB

(57) **Zusammenfassung**

Die Erfindung betrifft ein Verfahren zum Ermitteln einer Zellkonzentration einer Dosierflüssigkeit unter Verwendung einer Dosiervorrichtung mit einem Fluidkanal, einem Ventil (110), einer Druckquelle (140), einem ersten Dosierflüssigkeitsreservoir (160), in dem eine Dosierflüssigkeit aufgenommen ist, einer Verarbeitungseinrichtung, und einem dem Fluidkanal zugeordneten Fluidkanaldrucksensor (151), wobei der Fluidkanal einen ersten Fluidkanalabschnitt (121) und einen fluidisch mit dem ersten Fluidkanalabschnitt (121) verbundenen zweiten Fluidkanalabschnitt (122) aufweist, wobei das Ventil (110) ein Ventilglied (112) aufweist, das zwischen dem ersten und dem zweiten Fluidkanalabschnitt (122) angeordnet ist, und das in eine Offenstellung (113), in der die fluidische Verbindung des ersten Fluidkanalabschnitts (121) mit dem zweiten Fluidkanalabschnitt (122) freigegeben ist, und in eine Schließstellung (114) bewegt werden kann, in der die fluidische Verbindung des ersten Fluidkanalabschnitts (121) mit dem zweiten Fluidkanalabschnitt (122) geschlossen ist, wobei das erste Dosierflüssigkeitsreservoir (160) fluidisch mit dem ersten Fluidkanalabschnitt (121) verbunden ist, wobei die Dosierflüssigkeit biologische Zellen aufweist.

## Beschreibung

Die Erfindung betrifft ein Verfahren zum Ermitteln einer Zellkonzentration einer Dosierflüssigkeit, eine Dosiervorrichtung und einen Bioreaktor.

Bei bekannten Verfahren zum Ermitteln einer Zellkonzentration einer biologische Zellen enthaltenden Flüssigkeit werden optische Sensoren verwendet. Die zu untersuchende Flüssigkeit wird auf einen Probenträger aufgebracht. Anschließend wird der Probenträger mittels eines Mikroskops mikroskopisch untersucht. Gegebenenfalls wird hierbei zur Erhöhung des Kontrastes beziehungsweise Verbesserung der Sichtbarkeit der biologischen Zellen zusätzlich ein Kontrastmittel auf den Probenträger aufgebracht. Die mikroskopische Untersuchung umfasst eine optische Auswertung eines mit dem Mikroskop aufgenommenen Bildes dahingehend, dass biologische Zellen in festgelegten Flächenbereichen der Probe beziehungsweise des aufgenommenen Bild ausgezählt werden. Anhand des Zählergebnisses wird dann eine Zellkonzentration extrapoliert. Gemäß der vorgenannten Messmethode erfolgt eine Handhabung einer entsprechenden Flüssigkeitsprobe außerhalb eines für ein eigentliches biotechnologisches Verfahren verwendeten Reservoirs.

Aufgabe der vorliegenden Erfindung ist es, auf einfache, schnelle und zuverlässige Weise eine Zellkonzentration einer Dosierflüssigkeit zu ermitteln.

Die Aufgabe wird durch ein Verfahren nach Anspruch 1 und eine Dosiervorrichtung nach Anspruch 15 gelöst.

Bei einem erfindungsgemäßen Verfahren zum Ermitteln einer Zellkonzentration einer Dosierflüssigkeit wird eine Dosiervorrichtung verwendet, die einen Fluidkanal, ein Ventil, eine Druckquelle, ein erstes Dosierflüssigkeitsreservoir, in dem eine Dosierflüssigkeit aufgenommen ist, eine Verarbeitungseinrichtung, und einen dem Fluidkanal zugeordneten Fluidkanaldrucksensor aufweist, wobei der Fluidkanal einen ersten Fluidkanalabschnitt und einen fluidisch mit dem ersten Fluidkanalabschnitt verbundenen zweiten Fluidkanalabschnitt aufweist, wobei das Ventil ein Ventilglied aufweist, das zwischen dem ersten und dem zweiten Fluidkanalabschnitt angeordnet ist, und das in eine Offenstellung, in der die fluidische Verbindung des ersten Fluidkanalabschnitts mit dem zweiten Fluidkanalabschnitt freigegeben ist, und in eine Schließstellung bewegt werden kann, in der die fluidische Verbindung des ersten Fluidkanalabschnitts mit dem zweiten Fluidkanalabschnitt geschlossen ist, wobei das erste Dosierflüssigkeitsreservoir fluidisch mit dem ersten Fluidkanalabschnitt verbunden ist, wobei die Dosierflüssigkeit biologische Zellen aufweist.

Das erfindungsgemäße Verfahren umfasst die Schritte: Beaufschlagen des ersten Dosierflüssigkeitsreservoirs mit einem Druckfluid mittels der Druckquelle, sodass die Dosierflüssigkeit durch Druckbeaufschlagung in dem ersten Fluidkanalabschnitt bewegt wird, Bewegen des Ventilgliedes zu Beginn einer Dosierzeit von der Schließstellung in die Offenstellung, sodass die Dosierflüssigkeit in den zweiten Fluidkanalabschnitt bewegt wird und aus dem zweiten Fluidkanalabschnitt ausströmt, Bewegen des Ventilgliedes zum Ende der Dosierzeit von der Offenstellung in die Schließstellung, Ermitteln eines Fluidruckverlaufs während der Dosierzeit und/oder nach der Dosierzeit mit dem Fluidkanaldrucksensor, wobei die Verarbeitungseinrichtung den Fluiddruckverlauf verarbeitet, um eine Zellkonzentration der Dosierflüssigkeit zu ermitteln.

In dem Fluidkanal wird die Dosierflüssigkeit bewegt. Die Nummerierung der Fluidkanalabschnitte entspricht der Richtung der Bewegung der Dosierflüssigkeit innerhalb des Fluidkanals während des Dosiervorgangs, das heißt ausgehend von dem ersten Dosierflüssigkeitsreservoir. Dementsprechend tritt die Dosierflüssigkeit über den ersten Fluidkanalabschnitt in den Fluidkanal ein und strömt über den zweiten Fluidkanalabschnitt aus dem Fluidkanal aus.

Die Bewegung der Dosierflüssigkeit in dem Fluidkanal wird durch das Druckfluid initiiert, mit dem auf die in dem ersten Dosierflüssigkeitsreservoir aufgenommene Dosierflüssigkeit ein Druck ausgeübt wird. Dadurch resultiert gegenüber einer die Dosiervorrichtung umgebenden Umgebung ein Überdruck in dem ersten Dosierflüssigkeitsreservoir, der in Richtung des zweiten Fluidkanalabschnitts abgebaut wird. Das erste Dosierflüssigkeitsreservoir weist in einem Betriebszustand zumindest eine erste Öffnung auf, durch die der erste Fluidkanalabschnitt aus dem ersten Dosierflüssigkeitsreservoir herausragt. Alternativ oder in Ergänzung ist der erste Fluidkanalabschnitt einstückig an dem ersten Dosierflüssigkeitsreservoir angeformt, sodass der erste Fluidkanalabschnitt an der ersten Öffnung von dem ersten Dosierflüssigkeitsreservoir abragt.

Auf der dem zweiten Fluidkanalabschnitt abgewandten Seite des ersten Fluidkanalabschnitts taucht der erste Fluidkanalabschnitt in die Dosierflüssigkeit ein. Auf diese Weise kann gewährleistet werden, dass die Dosierflüssigkeit bei Vorliegen eines Überdrucks im Dosierflüssigkeitsreservoir in den Fluidkanal einströmt.

Mittels der Druckquelle wird das erste Dosierflüssigkeitsreservoir mit dem Druckfluid beaufschlagt. Dadurch wird das erste Dosierflüssigkeitsreservoir mit einem entsprechenden Druck beaufschlagt. Als Druckfluid kann ein Gas, also ein kompressibles Fluid, oder eine Flüssigkeit, also ein inkompressibles Fluid, verwendet werden. Wird als Druckfluid ein Gas verwendet, insbesondere Druckluft, kann das erste Dosierflüssigkeitsreservoir eine zweite Öffnung aufweisen, über die das als Gas ausgebildete Druckfluid in das erste Dosierflüssigkeitsreservoir eingespeist wird. Bevorzugt ist an der zweiten Öffnung ein fluidisch mit der Druckquelle verbundener Druckfluidkanal angeschlossen, insbesondere einstückig an dem erste Dosierflüssigkeitsreservoir angeformt.

Das erste Dosierflüssigkeitsreservoir kann dazu ausgebildet sein, dass für eine entsprechende Beaufschlagung Druckfluid in das erste Dosierflüssigkeitsreservoir eingeleitet wird. Dies ist insbesondere dann vorteilhaft, wenn als Druckfluid ein Gas verwendet wird, da hierbei eine Vermischung des Druckfluids mit der Dosierflüssigkeit prinzipbedingt nicht möglich ist. Bevorzugt weist das erste Dosierflüssigkeitsreservoir, wenn für eine entsprechende Beaufschlagung Druckfluid in das erste Dosierflüssigkeitsreservoir eingeleitet wird, eine starre Außenwandung auf.

Alternativ kann das erste Dosierflüssigkeitsreservoir dazu ausgebildet sein, dass für eine entsprechende Beaufschlagung Druckfluid von außen auf das erste Dosierflüssigkeitsreservoir aufgeprägt wird. Dies ist insbesondere dann vorteilhaft, wenn als Druckfluid eine Flüssigkeit verwendet wird, da hierbei eine Vermischung des Druckfluids mit der Dosierflüssigkeit prinzipbedingt zwar möglich ist, jedoch unter Berücksichtigung des Vorstehenden verhindert werden kann. Bevorzugt weist das erste Dosierflüssigkeitsreservoir, wenn für eine entsprechende Beaufschlagung Druckfluid von außen auf das erste Dosierflüssigkeitsreservoir aufgeprägt wird, eine flexible Außenwandung auf.

Im Sinne der vorliegenden Erfindung wird der Fluidkanal in Fluidkanalabschnitte unterteilt, um die unterschiedlichen Funktionen beziehungsweise Bereiche des Fluidkanals eindeutig bezeichnen und zuordnen zu können. Vorzugsweise sind der erste und/oder der zweite Fluidkanalabschnitt schlauchartig ausgebildet und ferner vorzugsweise mittels einer Schlauchkupplung mit dem Ventil verbunden.

Befindet sich das Ventilglied in der Schließstellung, sperrt das Ventilglied den Fluidkanal fluiddicht ab, sodass ein Übertritt der Dosierflüssigkeit von dem ersten Fluidkanalabschnitt zu dem zweiten Fluidkanalabschnitt verhindert wird. Damit wird auch verhindert, dass von dem ersten Dosierflüssigkeitsreservoir kommende Dosierflüssigkeit aus dem zweiten Fluidkanalabschnitt ausströmt. Befindet sich das Ventilglied in der Offenstellung, gibt das Ventilglied den Fluidkanal fluidisch frei, sodass Dosierflüssigkeit von dem ersten Fluidkanalabschnitt zu dem zweiten Fluidkanalabschnitt übertreten kann.

Die Dosierflüssigkeit weist biologische Zellen auf. Als biologische Zelle wird eine lebende Einheit eines Organismus bezeichnet. Die Dosierflüssigkeit wird insbesondere im Bereich der Biotechnologie eingesetzt, beispielsweise zu Therapiezwecken, wie Leukämie-Therapie, Herstellung von Geweben, wie künstlichen Organen, aber auch bei Brau- oder Gärprozessen, wie dem Bierbrauen unter Verwendung von Hefezellen. Als biologische Zellen kommen also insbesondere Säugerzellen, wie Blut- oder Stammzellen, ausdifferenzierte Zellen aus Geweben und auch natürliche oder gentechnisch veränderte Mikroorganismen, die in der Produktion von Biomolekülen und Pharmazeutika oder Fermentationsprozessen eingesetzt werden, in Betracht. Die Dosierflüssigkeit ist insbesondere eine wässrige, also niedrigviskose, insbesondere wasserbasierte oder organische, beispielsweise alkoholbasierte, Flüssigkeit oder ein entsprechendes Flüssigkeitsgemisch. Erfindungsgemäß wird die Zellkonzentration der Dosierflüssigkeit ermittelt. Die Zellkonzentration der Dosierflüssigkeit entspricht der Anzahl von biologischen Zellen in einem definierten Volumen der Dosierflüssigkeit. Je mehr biologische Zellen in dem definierten Volumen vorliegen, desto höher ist die Zellkonzentration der Dosierflüssigkeit. Je weniger biologische Zellen in dem definierten Volumen vorliegen, desto geringer ist die Zellkonzentration der Dosierflüssigkeit.

Vorzugsweise ist der Fluidkanaldrucksensor als Relativdrucksensor ausgebildet. Somit wird der Fluiddruckverlauf beziehungsweise Fluiddruck unmittelbar als Relativdruck gegenüber der Atmosphäre gemessen, das heißt als Fluidrelativdruck. Ferner vorzugsweise ist der Fluidkanaldrucksensor als piezoresistiver Drucksensor ausgebildet. Alternativ ist der Fluidkanaldrucksensor als piezoelektrischer oder kapazitiver Drucksensor ausgebildet. Bevorzugt ist der Fluidkanaldrucksensor dem ersten Fluidkanalabschnitt zugeordnet.

Wird als Druckfluid Druckluft verwendet, ist die Druckquelle vorzugsweise als dezentraler Druckluftgenerator ausgebildet, sodass die Druckquelle unmittelbar dem ersten Dosierflüssigkeitsreservoir zugeordnet sein kann. Eine derart ausgebildete Druckquelle weist einen Kompressor, eine Spannungsversorgung und insbesondere eine proportionale Druckregelung auf.

Vorzugsweise ist zwischen der Druckquelle und dem ersten Dosierflüssigkeitsreservoir ein Druckregelventil, insbesondere ein Proportional-Druckregelventil angeordnet, mit dem der Druck, mit dem das erste Dosierflüssigkeitsreservoir beaufschlagt wird, geregelt werden kann. Vorzugsweise ist das Druckregelventil unmittelbar vor dem, besonders bevorzugt in dem ersten Dosierflüssigkeitsreservoir angeordnet. Dies ist insbesondere vorteilhaft, wenn als Druckfluid ein Gas, insbesondere Druckluft, verwendet wird. Hierbei kann der Druck des kompressiblen Gases in unmittelbarer Nähe des Ortes geregelt werden, an dem mit dem Druckfluid die Dosierflüssigkeit in den Fluidkanal bewegt wird. Auf diese Weise kann eine schnelle, das heißt hochfrequente Regelung des Druckes des Druckfluides erfolgen, mit der die Dosierflüssigkeit in den Fluidkanal bewegt wird. Dadurch wird eine kontrollierte Bewegung der Dosierflüssigkeit in dem Fluidkanal und ein kontrolliertes Ausströmen der Dosierflüssigkeit aus dem zweiten Fluidkanalabschnitt ermöglicht. Insbesondere kann hierdurch erreicht werden, dass zum Ende des Dosiervorgangs hin ein Tropfenabriss der Dosierflüssigkeit an dem zweiten Fluidkanalabschnitt in gewünschter Weise erfolgt.

Die Verarbeitungseinrichtung ist dazu eingerichtet, das erfindungsgemäße Verfahren auszuführen. Hierfür weist die Verarbeitungseinrichtung vorzugsweise zumindest eine elektrische Schaltung, insbesondere einen Mikroprozessor oder Mikrocontroller, auf, auf der ein Computerprogrammcode zur Ausführung des erfindungsgemäßen Verfahrens hinterlegt ist und/oder mit der der Computerprogrammcode zur Ausführung des erfindungsgemäßen Verfahrens ausführbar ist. Ferner vorzugsweise ist die Verarbeitungseinrichtung dazu eingerichtet, den vom Fluidkanaldrucksensor erfassten Fluiddruckverlauf zu erfassen und, insbesondere in der Verarbeitungseinrichtung, zu hinterlegen.

Das erfindungsgemäße Verfahren ist beispielsweise zur Verwendung in Umgebungen mit hohem Automationsgrad angedacht. Indem erfindungsgemäß vorgesehen ist, mit dem Fluiddruckverlauf die Zellkonzentration der Dosierflüssigkeit zu ermitteln, kann zur Ermittlung der Zellkonzentration der Dosierflüssigkeit auf weitere Messmittel verzichtet werden, insbesondere auf optische Sensoren beziehungsweise Mikroskope.

Als Dosierzeit wird eine zeitliche Differenz beziehungsweise ein Zeitintervall zwischen dem Zeitpunkt zu Beginn des Dosierens und dem Zeitpunkt zum Ende des Dosierens bezeichnet. Als Zeitpunkt zu Beginn des Dosierens gilt der Zeitpunkt, zu dem eine fluidische Verbindung zwischen dem ersten Fluidkanalabschnitt und dem zweiten Fluidkanalabschnitt besteht, nachdem die Bewegung des Ventilgliedes von der Schließstellung in die Offenstellung initiiert wurde. Das ist beispielsweise bei Verwendung eines als Membranventil ausgebildeten Ventils der Zeitpunkt, zu dem die Membran aufgrund der Bewegung des Ventilglieds von einem Ventilsitz abhebt. Als Zeitpunkt zum Ende des Dosierens gilt der Zeitpunkt, zu dem die fluidische Verbindung zwischen dem ersten Fluidkanalabschnitt und dem zweiten Fluidkanalabschnitt unterbrochen ist, nachdem die Bewegung des Ventilgliedes von der Offenstellung in die Schließstellung initiiert wurde. Das ist beispielsweise bei Verwendung eines als Membranventil ausgebildeten Ventils der Zeitpunkt, zu dem die Membran wieder abdichtend auf dem Ventilsitz aufliegt.

Der Fluiddruckverlauf ist ein zeitlicher Verlauf des mit dem Fluidkanaldrucksensors erfassten Fluiddruckes. Der Fluiddruckverlauf kann zu verschiedenen Zeitpunkten verschiedene Fluiddrücke umfassen, insbesondere steigt der Fluiddruckverlauf bis zu einem als Maximum ausgebildeten Extremum an beziehungsweise fällt bis zu einem als Minimum ausgebildeten Extremum, um danach wieder abzufallen beziehungsweise anzusteigen.

Die Erfinder sind zu der überraschenden Erkenntnis gelangt, dass der Fluiddruckverlauf von einer Viskosität der Dosierflüssigkeit beeinflusst wird und dass bei Kenntnis des Fluiddruckverlaufs Rückschlüsse auf die Viskosität der Dosierflüssigkeit gezogen werden können. Die Erfinder sind weiterhin zu der überraschenden Erkenntnis gelangt, dass die Zellkonzentration der Dosierflüssigkeit deren Viskosität beeinflusst und somit von dem Fluiddruckverlauf Rückschlüsse auf die Zellkonzentration der Dosierflüssigkeit gezogen werden können.

Bei der Viskosität von Flüssigkeiten wird zwischen der dynamischen Viskosität und der kinematischen Viskosität unterschieden, wobei zwischen diesen beiden ein proportionaler Zusammenhang besteht. Insbesondere ergibt sich die kinematische Viskosität einer Flüssigkeit als Quotient aus der dynamischen Viskosität der Flüssigkeit als Dividend und der Dichte der Flüssigkeit als Divisor. Mit anderen Worten ergibt sich die dynamische Viskosität der Flüssigkeit als Produkt aus den Faktoren kinematische Viskosität der Flüssigkeit und Dichte der Flüssigkeit. Steigt die kinematische Viskosität der Flüssigkeit, steigt auch die dynamische Viskosität der Flüssigkeit. Sinkt die kinematische Viskosität der Flüssigkeit, sinkt auch die dynamische Viskosität der Flüssigkeit.

Potentiell beeinflussen die im Folgenden dargelegten physikalischen und biologischen Wirkzusammenhänge die Viskosität der Dosierflüssigkeit. Dabei besteht eine Wechselwirkung zwischen den einzelnen Aspekten dahingehend, dass beispielsweise ein die Viskosität erhöhender Einfluss eines Aspektes durch einen die Viskosität verringernden Einfluss eines anderen Aspektes ausgeglichen werden kann oder dass ein die Viskosität erhöhender Einfluss eines Aspektes durch einen ebenfalls die Viskosität erhöhenden Einflusses eines anderen Aspektes verstärkt werden kann.

Die biologischen Zellen nehmen Platz in der Dosierflüssigkeit ein und behindern den Fluss von Flüssigkeitsmolekülen, die die biologischen Zellen umgeben. Je mehr biologische Zellen die Dosierflüssigkeit aufweist, desto größer ist die Viskosität der Dosierflüssigkeit. Je weniger biologische Zellen die Dosierflüssigkeit aufweist, desto geringer ist die Viskosität der Dosierflüssigkeit. Dementsprechend ist unter der Annahme einer gleichbleibenden durchschnittlichen Größe der biologischen Zellen die Viskosität einer Dosierflüssigkeit mit einer hohen Zellkonzentration größer als die Viskosität einer Dosierflüssigkeit mit einer geringen Zellkonzentration.

Die biologischen Zellen weisen je nach Typ eine mehr oder weniger ausgeprägte Verformbarkeit beziehungsweise Deformierbarkeit beziehungsweise Flexibilität auf. Bei einer Bewegung der Dosierflüssigkeit werden auch die in der Dosierflüssigkeit vorhandenen biologischen Zellen relativ zu der Dosierflüssigkeit bewegt, wodurch die bewegten biologischen Zellen verformt beziehungsweise deformiert werden. Die Verformung beziehungsweise Deformation kann zumindest teilweise elastisch sein. Durch diese Verformung beziehungsweise Deformation wird Bewegungsenergie der Dosierflüssigkeit in entsprechende Verformungsenergie umgewandelt, sodass ein der Bewegung der Dosierflüssigkeit entgegengesetzter Widerstand resultiert. Dieser Widerstand trägt zur Viskosität der Dosierflüssigkeit bei. Dabei ist die Viskosität der Dosierflüssigkeit umso höher, je mehr verformungsbedingter Widerstand besteht, das heißt umso höher, je verformbarer beziehungsweise deformierbarer die biologischen Zellen sind. Bei geringerer Verformbarkeit beziehungsweise Deformierbarkeit der biologischen Zellen resultiert dementsprechend eine geringere Viskosität der Dosierflüssigkeit.

Zudem kann die Viskosität durch eine Interaktion der biologischen Zellen untereinander beeinflusst werden, beispielsweise durch Adhäsion. Je dichter die biologischen Zellen vorliegen, desto stärker interagieren die biologischen Zellen untereinander und desto stärker ist beispielsweise eine Summe der zwischen den biologischen Zellen ausgeprägten Adhäsionskräfte. Hierdurch steigt die Viskosität der Dosierflüssigkeit. Je geringer die Interaktion zwischen den biologischen Zellen ausgeprägt ist, beispielsweise weil die biologischen Zellen weniger dicht vorliegen, desto geringer ist die Viskosität der Dosierflüssigkeit. Dementsprechend wird bei einer Dosierflüssigkeit mit einer hohen Zellkonzentration im Vergleich zu einer Dosierflüssigkeit mit einer geringen Zellkonzentration die Viskosität zusätzlich durch eine erhöhte Interaktion der biologischen Zellen untereinander erhöht.

Außerdem weisen die biologischen Zellen eine höhere Dichte auf als die Dosierflüssigkeit ohne biologische Zellen. Dementsprechend weist eine Dosierflüssigkeit, die viele biologische Zellen aufweist, eine höhere Dichte auf als eine Dosierflüssigkeit, die wenige biologische Zellen aufweist. Auch in dieser Hinsicht wird durch mehr in einer Dosierflüssigkeit vorhandene biologische Zellen die Viskosität dieser Dosierflüssigkeit erhöht.

Unter Berücksichtigung des Vorstehenden ist grundsätzlich davon auszugehen, dass eine Dosierflüssigkeit mit einer hohen Zellkonzentration eine größere Viskosität beziehungsweise dynamische Viskosität aufweist als eine Dosierflüssigkeit mit einer geringen Zellkonzentration.

Vorzugsweise weist der zweite Fluidkanalabschnitt eine Dosierspitze auf, wobei die Dosierspitze an einem Ende des zweiten Fluidkanalabschnittes angeordnet ist, das dem ersten Fluidkanalabschnitt abgewandt ist, wobei die Dosierflüssigkeit in Folge dessen, dass die Dosierflüssigkeit aus dem zweiten Fluidkanalabschnitt ausströmt, aus der Dosierspitze ausströmt. Der zweite Fluidkanalabschnitt und die Dosierspitze können einstückig ausgebildet sein. Alternativ sind der zweite Fluidkanalabschnitt und die Dosierspitze getrennt voneinander ausgebildet und liegen dementsprechend als separate Komponenten vor.

Vorzugsweise verarbeitet die Verarbeitungseinrichtung zusätzlich einen Geometriekennwert der Dosierspitze, um die Zellkonzentration der Dosierflüssigkeit zu ermitteln. Die Dosierspitze weist eine bekannte Innengeometrie auf, die mit der Dosierflüssigkeit in Kontakt steht. Anhand von Abmessungen der Innengeometrie der Dosierspitze wird der Geometriekennwert der Dosierspitze bestimmt. Bevorzugt sind die innengeometrischen Abmessungen der Dosierspitze innerhalb eines gewissen Toleranzbereiches bekannt, sodass der Geometriekennwert der Dosierspitze ohne ein vorheriges separates Ausmessen der Innengeometrie der Dosierspitze bestimmbar ist. Vorzugsweise ist die Innengeometrie der Dosierspitze rotationssymmetrisch, gegebenenfalls zumindest abschnittsweise kreiszylindrisch und zu einer Dosieröffnung hin kegelförmig verjüngt ausgebildet. Ferner vorzugsweise wird bei der Bestimmung des Geometriekennwerts der Dosierspitze der Radius und/oder die Länge des Hohlraums der Dosierspitze berücksichtigt. Besonders bevorzugt ergibt sich der Geometriekennwert der Dosierspitze als Quotient, aus einem Divisor und einem Dividenden, wobei der Dividend der vierten Potenz des Radius des Hohlraums der Dosierspitze entspricht und wobei der Divisor der Länge des Hohlraums der Dosierspitze entspricht.

Grundsätzlich wirken alle Komponenten der Dosiervorrichtung, durch die die Dosierflüssigkeit bewegt wird, reibungsbedingt und/oder bedingt durch Querschnittsänderungen als Drossel. Hierbei ist insbesondere neben der Dosierspitze auch der Fluidkanal von Bedeutung. Jedoch setzt die typischerweise zur Dosieröffnung spitz zulaufende Dosierspitze der Bewegung der Dosierflüssigkeit vorzugsweise einen zumindest doppelt so großen Widerstand entgegen wie die anderen Komponenten der Dosiervorrichtung. Somit wird die Dosierspitze bewusst als Drossel eingesetzt, während bei den anderen Komponenten die Drosselwirkung prinzipbedingt zu akzeptieren ist. Die Drosselwirkung der Dosierspitze kann durch deren Innengeometrie beeinflusst werden. Beispielsweise kann durch Vergrößerung der Länge des Hohlraums der Dosierspitze und/oder durch Verringerung des Radius des Hohlraums der Dosierspitze der der Dosierflüssigkeit entgegengebrachte Widerstand erhöht und damit die Drosselwirkung verstärkt werden. Zudem kann beispielsweise durch Verringerung der Länge des Hohlraums der Dosierspitze und/oder durch Erhöhung des Radius des Hohlraums der Dosierspitze der der Dosierflüssigkeit entgegengebrachte Widerstand verringert und damit die Drosselwirkung abgeschwächt werden.

Vorzugsweise wird unmittelbar nachdem das Ventilglied von der Schließstellung in die Offenstellung bewegt wurde oder unmittelbar nachdem das Ventilglied von der Offenstellung in die Schließstellung bewegt wurde, mit dem Fluidkanaldrucksensor ein Fluiddruckschwingungsverlauf bestimmt, wobei auf Basis des Fluiddruckschwingungsverlaufs die Zellkonzentration der Dosierflüssigkeit ermittelt wird.

Durch die Bewegung des Ventilgliedes wird die Bewegung der Dosierflüssigkeit in dem Fluidkanal beeinflusst. Nach dem Bewegen des Ventilgliedes von der Schließstellung in die Offenstellung kommt es im zweiten Fluidkanalabschnitt zu einem abrupten Druckanstieg, durch den eine Druckwelle im zweiten Fluidkanalabschnitt hervorgerufen wird. Stößt diese Druckwelle auf ein Hindernis, insbesondere auf die als Drossel wirkende Dosierspitze, wird die Druckwelle an dem Hindernis zumindest teilweise reflektiert, woraus eine Druckschwingung resultiert. Der zeitliche Verlauf dieser Druckschwingung wird in Bezug auf den Fluiddruck als Fluiddruckschwingungsverlauf bezeichnet. Der Fluiddruckschwingungsverlauf wird vom Fluid-drucksensor bestimmt und ist vorzugsweise ein zeitbezogener Teil des Fluiddruckverlaufs, der insbesondere unmittelbar nach dem Bewegen des Ventilgliedes von der Schließstellung in die Offenstellung, das heißt zu Beginn der Dosierzeit liegt oder unmittelbar nach dem Bewegen des Ventilgliedes von der Offenstellung in die Schließstellung, das heißt zum Ende der Dosierzeit liegt.

In gleicher Weise tritt eine Druckschwingung auf, wenn das Ventilglied von der Offenstellung in die Schließstellung bewegt wird. In diesem Fall kommt es zu einem abrupten Druckabfall. In dem dem ersten Fluidkanalabschnitt zugewandten Bereich des zweiten Fluidkanalabschnitts tritt dann zumindest kurzzeitig ein geringerer Druck auf als in dem restlichen Bereich des zweiten Fluidkanalabschnitts. Dieser Druckunterschied führt zu einem Druckausgleich, der entgegen der während des Dosierens ausgebildeten Bewegungsrichtung der Dosierflüssigkeit gerichtet ist.

Dementsprechend kann der Fluiddruckschwingungsverlauf sowohl nach dem Bewegen des Ventilgliedes von der Schließstellung in die Offenstellung als auch nach dem Bewegen des Ventilgliedes von der Offenstellung in die Schließstellung bestimmt werden.

Der Fluiddruckschwingungsverlauf ist von der dynamischen und/oder kinematischen Viskosität der Dosierflüssigkeit abhängig. Die kinematische Viskosität ist ein Maß dafür, wie die Dosierflüssigkeit unter Schwerkrafteinfluss fließt. Die dynamische Viskosität ist ein Maß dafür, welchen Widerstand die Dosierflüssigkeit gegen Verformung aufbringt. Da der Druckschwingungsverlauf wie vorstehend beschrieben aufgrund einer Beeinflussung der Bewegung der Dosierflüssigkeit resultiert, übt eine Dosierflüssigkeit mit einer hohen dynamischen Viskosität einen größeren Widerstand auf die Bewegungsbeeinflussung aus als eine Dosierflüssigkeit mit einer geringen dynamischen Viskosität. Somit wird der Druckverlauf beziehungsweise Fluiddruckschwingungsverlauf einer Dosierflüssigkeit mit einer hohen dynamischen Viskosität und damit hohen Zellkonzentration weniger stark beeinflusst als der Druckverlauf beziehungsweise Fluiddruckschwingungsverlauf einer Dosierflüssigkeit mit einer geringen dynamischen Viskosität und damit geringen Zellkonzentration.

Das kann sich zum einen darin äußern, dass bei einer Dosierflüssigkeit mit einer hohen dynamischen Viskosität geringere Druckamplituden erreicht werden als bei einer Dosierflüssigkeit mit einer geringen dynamischen Viskosität. Dies liegt darin begründet, dass bei einer Dosierflüssigkeit mit einer hohen dynamischen Viskosität eine stärkere Dämpfung der mit den Druckamplituden einhergehenden Druckschwingung. Diese Art des Dämpfens wird auch als viskoses Dämpfen bezeichnet. Zum anderen kann sich das darin äußern, dass bei einer Dosierflüssigkeit mit einer hohen dynamischen Viskosität die Frequenz der Druckamplituden größer ist als bei einer Dosierflüssigkeit mit einer geringen dynamischen Viskosität. Außerdem kann sich das darin äußern, dass bei einer Dosierflüssigkeit mit einer hohen dynamischen Viskosität die Abklingrate der Amplituden aufeinanderfolgender Druckextremwerte größer ist, das heißt die Druckschwingung stärker gedämpft wird als bei einer Dosierflüssigkeit mit einer geringen dynamischen Viskosität.

Unter Berücksichtigung der vorstehend beschriebenen Zusammenhänge zwischen dem Fluiddruckschwingungsverlauf und der dynamischen und/oder kinematischen Viskosität der Dosierflüssigkeit sowie der Viskosität der Dosierflüssigkeit und den biologischen und/oder physikalischen Einflüssen der biologischen Zellen der Dosierflüssigkeit kann von dem Fluiddruckschwingungsverlauf beziehungsweise von von dem Fluiddruckschwingungsverlauf abgeleiteten Kennwerten auf die Zellkonzentration der Dosierflüssigkeit geschlossen werden.

Vorzugsweise wird die Zellkonzentration der Dosierflüssigkeit mit zumindest einem Kennwert aus der Gruppe: Amplitude des ersten Extremwerts, Amplitude des zweiten Extremwerts, Amplitude des dritten Extremwerts, Zeitdifferenz zwischen dem ersten Extremwert und/oder dem zweiten Extremwert und/oder dem dritten Extremwert, Abklingrate der Amplitude zumindest zweier aufeinanderfolgender Extremwerte, ermittelt, der anhand des Fluiddruckschwingungsverlaufs bestimmt wird. Unter Berücksichtigung der vorstehend beschriebenen Mechanismen lassen sich von den vorgenannten amplitudenbezogenen Kennwerten Rückschlüsse auf die Viskosität der Dosierflüssigkeit und damit auf die Zellkonzentration der Dosierflüssigkeit ziehen.

Als Amplitude wird eine Differenz zwischen dem betreffenden Extremum und einem Normaldruck bezeichnet, insbesondere die Differenz zwischen dem betreffenden Extremum und einem sich bei konstantem Schließen beziehungsweise konstantem Öffnen einstellendem Fluiddruck.

Die Nummerierung der amplitudenbezogenen Extremwerte folgt dem zeitlichen Auftreten der betreffenden Extremwerte, das heißt der erste Extremwert tritt zuerst auf, gefolgt von dem zweiten Extremwert. Anschließend tritt der dritte Extremwert auf. Es ist möglich, die Amplituden weiterer Extremwerte als Kennwerte zur Ermittlung der Zellkonzentration der Dosierflüssigkeit zu verwenden, beispielsweise die Amplitude des vierten, fünften, sechsten, siebten, achten, neunten und/oder zehnten Extremwerts. Die Extremwerte können jeweils als Maximum oder als Minimum ausgebildet sein.

Als zeitliche Abstände zwischen den Extremwerten können unter anderem der zeitliche Abstand zwischen dem ersten und dem zweiten Extremwert, der zeitliche Abstand zwischen dem ersten und dem dritten Extremwert und der zeitliche Abstand zwischen dem zweiten und dem dritten Extremwert verwendet werden. Sofern weitere Extremwerte bestimmt beziehungsweise berücksichtigt werden, können iterativ weitere zeitliche Abstände berücksichtigt werden, beispielsweise der zeitliche Abstand zwischen dem ersten und dem vierten Extremwert, der zeitliche Abstand zwischen dem zweiten und dem vierten Extremwert und der zeitliche Abstand zwischen dem dritten und dem vierten Extremwert und so weiter.

Die Abklingrate der Amplitude zumindest zweier aufeinanderfolgender Extremwerte ist ein Maß dafür, wie schnell die Amplitudenwerte aufeinanderfolgender Extremwerte absinken. Bei einer großen Abklingrate sinken die Amplitudenwerte stärker beziehungsweise schneller ab als bei einer geringen Abklingrate.

Vorzugsweise wird vor der Ermittlung der Zellkonzentration der Dosierflüssigkeit die Verarbeitungseinrichtung parametriert, indem für zumindest eine Testflüssigkeit mit bekannter Zellkonzentration die folgenden Schritte durchgeführt werden: Bewegen der Testflüssigkeit in dem Fluidkanal mittels Bewegen des Ventilgliedes von der Schließstellung zu der Offenstellung und anschließend Bewegen des Ventilgliedes von der Offenstellung zu der Schließstellung, wobei in Zusammenhang mit dem Bewegen der Testflüssigkeit in dem Fluidkanal unmittelbar nachdem das Ventilglied von der Schließstellung in die Offenstellung bewegt wurde oder unmittelbar nachdem das Ventilglied von der Offenstellung in die Schließstellung bewegt wurde, mit dem Fluidkanaldrucksensor ein Test-Fluiddruckschwingungsverlauf bestimmt wird, wobei auf Basis des Test-Fluiddruckschwingungsverlaufs die Zellkonzentration der Testflüssigkeit ermittelt wird, wobei die Zellkonzentration der Testflüssigkeit mit zumindest einem Kennwert aus der Gruppe: Amplitude des ersten Extremwerts, Amplitude des zweiten Extremwerts, Amplitude des dritten Extremwerts, Zeitdifferenz zwischen dem ersten Extremwert und/oder dem zweiten Extremwert und/oder dem dritten Extremwert, Abklingrate der Amplitude zumindest zweier aufeinanderfolgender Extremwerte, ermittelt wird, der anhand des Test-Fluiddruckschwingungsverlaufs bestimmt wird, wobei anschließend zumindest eine Zellkonzentrationsklasse unter Berücksichtigung der ermittelten Zellkonzentration der zumindest einen Testflüssigkeit ermittelt wird, wobei bei der anschließenden Ermittlung der Zellkonzentration bei entsprechender Übereinstimmung der Dosierflüssigkeit die zumindest eine Zellkonzentrationsklasse zugeordnet wird, wobei mit dieser zugeordneten zumindest einen Zellkonzentrationsklasse die Zellkonzentration der Dosierflüssigkeit ermittelt wird.

Indem die Zellkonzentration der zumindest einen Testflüssigkeit ermittelt wird und darauf basierend eine Zellkonzentrationsklasse ermittelt wird, kann auf die direkte Ermittlung der Zellkonzentration der Dosierflüssigkeit, beispielsweise auf Basis des Fluiddruckschwingungsverlaufs, verzichtet werden. Vielmehr wird die für die Ermittlung der Zellkonzentration der Dosierflüssigkeit gemäß der vorstehenden bevorzugten Fortbildung der Erfindung erforderliche zugeordnete Zellkonzentrationsklasse verwendet. Diese zugeordnete Zellkonzentrationsklasse kann, wenn sie einmal ermittelt und beispielsweise in der Verarbeitungseinrichtung hinterlegt ist, in Bezug auf zeitlich nachfolgende Dosiervorgänge an Stelle der Zellkonzentration der jeweiligen Dosierflüssigkeit treten. Wird auf die Zellkonzentration der Dosierflüssigkeit Bezug genommen, kann somit eine zugeordnete Zellkonzentrationsklasse gemeint sein aber auch eine beispielsweise auf Basis des Fluiddruckschwingungsverlaufs ohne Vorkenntnisse zu zumindest einer Zellkonzentrationsklasse ermittelte Zellkonzentration der Dosierflüssigkeit gemeint sein.

Bevorzugt ist die Zuordnung einer Zellkonzentrationsklasse zu der jeweiligen Dosierflüssigkeit weniger aufwendig als die Ermittlung einer betreffenden Zellkonzentration der Dosierflüssigkeit ohne Vorkenntnisse zu zumindest einer Zellkonzentrationsklasse. Insbesondere erfolgt die Zuordnung zu einer Zellkonzentrationsklasse anhand weniger amplitudenbezogener Kennwerte, gegebenenfalls anhand lediglich eines amplitudenbezogenen Kennwertes, während die Ermittlung der Zellkonzentration der betreffenden Testflüssigkeit anhand von mehr amplitudenbezogenen Kennwerten erfolgt.

Wird der Fluiddruckschwingungsverlauf bestimmt, nachdem das Ventilglied von der Offenstellung in die Schließstellung bewegt wurde, kann die Zeit zwischen der Bewegung des Ventilgliedes von der Schließstellung in die Offenstellung und der Bewegung des Ventilgliedes von der Offenstellung in die Schließstellung minimiert werden. Alternativ oder in Ergänzung kann als die Zeit zwischen der Bewegung des Ventilgliedes von der Schließstellung in die Offenstellung und der Bewegung des Ventilgliedes von der Offenstellung in die Schließstellung eine Testdosierzeit verwendet werden.

Bevorzugt wird bei der Parametrierung der Verarbeitungseinrichtung zusätzlich ein von der Druckquelle bereitgestellter maximaler Speisedruck berücksichtigt, der mit dem Fluidkanaldrucksensor bestimmt wird, während sich das Ventilglied in der Schließstellung befindet. Besonders bevorzugt werden mehrere verschiedene maximale Speisedrücke berücksichtigt. Hierdurch kann die Genauigkeit der Ermittlung der Zellkonzentration der Dosierflüssigkeit auch bei Verwendung verschiedener maximaler Speisedrücke erhöht werden.

Vorzugsweise wird die Verarbeitungseinrichtung mit mehreren Testflüssigkeiten mit jeweils bekannten, unterschiedlichen Zellkonzentrationen parametriert, wobei mehrere Zellkonzentrationsklassen unter Berücksichtigung der jeweils für die mehreren Testflüssigkeiten ermittelten Zellkonzentration ermittelt werden. Hierdurch kann die Genauigkeit der Ermittlung der Zellkonzentration der Dosierflüssigkeit erhöht werden. Die Zellkonzentrationsklasse kann auch als entsprechende Zellkonzentration mit einer gewissen Standardabweichung angesehen werden. Ferner vorzugsweise wird jeweils für eine Testflüssigkeit mehrfach eine Zellkonzentration ermittelt, wobei jeweils auf Basis der mehreren Zellkonzentrationen einer Testflüssigkeit die entsprechende Zellkonzentrationsklasse bestimmt wird.

Bevorzugt werden die für die Parametrierung der Verarbeitungseinrichtung verwendeten bekannten, unterschiedlichen Zellkonzentrationen mittels vorbekannter Messverfahren ermittelt, beispielsweise mittels dem vorgenannten mikroskopgestützten Zählverfahren.

Vorzugsweise werden die unterschiedlichen Viskositätsklassen unter Verwendung eines zellkonzentrationsbezogenen Modells für maschinelles Lernen ermittelt. Das zellkonzentrationsbezogene Modell für maschinelles Lernen ist ein Modell, in dem zumindest ein statistischer Lernalgorithmus hinterlegt ist und mit dem funktionale Zusammenhänge zwischen zumindest einer Zellkonzentration der Dosierflüssigkeit als Eingangsgröße sowie gegebenenfalls anderen Eingangsgrößen und zumindest einer Zellkonzentrationsklasse als Ausgangsgröße ermittelt werden können. Hierdurch kann darauf verzichtet werden, aufwendig Kenntnisse zu physikalischen Zusammenhängen betreffend die Dosierflüssigkeit und die Dosiervorrichtung zu erlangen, wodurch insbesondere die Parametrierung der Verarbeitungseinrichtung vereinfacht wird.

Bevorzugt wird das zellkonzentrationsbezogene Modell für maschinelles Lernen trainiert, nachdem die zumindest eine Zellkonzentrationsklasse ermittelt wurde. Besonders bevorzugt wird, nachdem das zellkonzentrationsbezogene Modell für maschinelles Lernen trainiert wurde, die Zellkonzentration der Dosierflüssigkeit unter Berücksichtigung des trainierten zellkonzentrationsbezogenen Modells für maschinelles Lernen ermittelt.

Vorzugsweise weist die Dosiervorrichtung ein zweites Dosierflüssigkeitsreservoir auf, wobei das zweite Dosierflüssigkeitsreservoir fluidisch mit dem zweiten Fluidkanalabschnitt verbunden ist, wobei die aus dem zweiten Fluidkanalabschnitt ausströmende Dosierflüssigkeit in das zweite Dosierflüssigkeitsreservoir einströmt. Hierdurch kann die aus dem zweiten Fluidkanalabschnitt ausströmende Dosierflüssigkeit, insbesondere unmittelbar, biotechnologisch weiterverwendet werden. Eines solche biotechnologische Weiterverwendung kann beispielsweise darin bestehen, dass die genannte Dosierflüssigkeit weiter kultiviert wird oder biologische Zellen aus der genannten Dosierflüssigkeit extrahiert werden.

Vorzugsweise besteht zwischen einem in dem ersten Dosierflüssigkeitsreservoir ausgebildeten ersten Innendruck und einem in dem zweiten Dosierflüssigkeitsreservoir ausgebildeten zweiten Innendruck ein Unterschied von zumindest 30 mbar, ferner vorzugsweise von zumindest 45 mbar und besonders bevorzugt von zumindest 60 mbar. Ferner vorzugsweise ist der erste Innendruck größer als der zweite Innendruck. Hierdurch kann zum einen besser gewährleistet werden, dass Dosierflüssigkeit von dem ersten Dosierflüssigkeitsreservoir hin zu dem zweiten Dosierflüssigkeitsreservoir bewegt wird. Zum anderen kann hierdurch besser gewährleistet werden, dass der Fluiddruckschwingungsverlauf in einem Umfang auftritt, in dem der Fluiddruckschwingungsverlauf gut bestimmbar ist.

Die zuvor formulierte Aufgabe wird auch durch einen Bioreaktor gelöst.

Vorzugsweise ist zumindest eines der beiden Dosierflüssigkeitsreservoirs Teil eines Bioreaktors, wobei die Dosierflüssigkeit ferner einen Nährstoff aufweist. Außerdem kann die Dosierflüssigkeit Wachstumsfaktoren und/oder eine Pufferlösung aufweisen. Die Pufferlösung kann zum Abpuffern beispielsweise des pH-Wertes der Dosierflüssigkeit dienen. Ferner vorzugsweise sind beide Dosierflüssigkeitsreservoirs Teil des Bioreaktors. Besonders bevorzugt ist die Dosiervorrichtung Teil des Bioreaktors. Der Bioreaktor ist eine Vorrichtung, mit der biologische Zellen kultiviert werden und mit dem eine biologisch aktive Umgebung hergestellt werden kann. So kann auf einfache Weise, insbesondere inline, ermittelt werden, wie der Fortschritt der Zellkultivierung ist. Als Nährstoff wird jegliche Substanz bezeichnet, die die biologischen Zellen verstoffwechseln können. Beispielsweise kann Glukose als Nährstoff verwendet werden. Bevorzugt ist der Bioreaktor dazu ausgebildet, die Aktivität beziehungsweise das Wachstum der in dem betreffenden Dosierflüssigkeitsreservoir aufgenommenen biologischen Zellen zu beeinflussen. Hierfür kann der Bioreaktor beispielsweise eine entsprechende Temperaturregelung, eine Nährstoffzufuhr, eine Prozessgaszufuhr, insbesondere eine Sauerstoff- beziehungsweise Luftzufuhr, und/oder ein Rührwerk aufweisen.

Vorzugsweise weist die Dosiervorrichtung ferner einen Nährstoffsensor auf, wobei mit dem Nährstoffsensor eine Nährstoffkonzentration der Dosierflüssigkeit ermittelt wird, wobei die Verarbeitungseinrichtung zusätzlich die Nährstoffkonzentration der Dosierflüssigkeit verarbeitet, um die Zellkonzentration der Dosierflüssigkeit zu ermitteln. Die Nährstoffkonzentration entspricht der Menge an Nährstoff in einem definierten Volumen der Dosierflüssigkeit. In der Dosierflüssigkeit vorhandener Nährstoff beziehungsweise vorhandene Nährstoffe und somit die Nährstoffkonzentration können die Viskosität der Dosierflüssigkeit und somit unter Berücksichtigung der vorstehenden Wirkzusammenhänge den Fluiddruckverlauf beeinflussen. Ist die Nährstoffkonzentration nicht bekannt, resultiert bei der Ermittlung der Zellkonzentration eine gewisse Ungenauigkeit. Damit der Einfluss der Nährstoffkonzentration und damit die in diesem Zusammenhang einhergehende Ungenauigkeit bei der Ermittlung der Zellkonzentration reduziert werden, ist es vorteilhaft, die Nährstoffkonzentration der Dosierflüssigkeit wie vorstehend beschrieben zu berücksichtigen.

Der Nährstoffsensor kann dem Fluidkanal zugeordnet sein und/oder zumindest einem der Dosierflüssigkeitsreservoirs, beispielsweise dem ersten Dosierflüssigkeitsreservoir und/oder dem zweiten Dosierflüssigkeitsreservoir. Der Nährstoffsensor kann als Glukosesensor ausgebildet sein. Die Dosiervorrichtung kann ferner einen Sauerstoffsensor, einen Kohlenstoffdioxidsensor, einen Elektrolytsensor (beispielsweise einen ionenselektiven Sensor) und/oder einen anderen Sensor aufweisen, mit dem extrazelluläre Bestandteile messbar sind.

Alternativ oder gemäß einer vorteilhaften Ergänzung ist der Fluidkanaldrucksensor derart angeordnet, dass der Fluidkanaldrucksensor die Probenflüssigkeit nicht unmittelbar berührt. Ferner vorzugsweise ist die Druckquelle als hydraulische Druckquelle ausgebildet und wird als Druckfluid eine Hydraulikflüssigkeit verwendet, wobei das erste Dosierflüssigkeitsreservoir von außerhalb mit dem Druckfluid mittels der Druckquelle beaufschlagt wird. Eine Beaufschlagung des ersten Dosierflüssigkeitsreservoir von außerhalb bedeutet, dass das Druckfluid nicht in Kontakt mit der Dosierflüssigkeit kommt.

Vorzugsweise weist die Dosiervorrichtung ferner einen dem ersten Dosierflüssigkeitsreservoir zugeordneten ersten Reservoirdrucksensor auf. Ferner vorzugsweise wird während der Ermittlung des Fluiddruckverlaufs mit dem ersten Reservoirdrucksensor ein in dem ersten Dosierflüssigkeitsreservoir ausgebildeter erster Innendruck ermittelt, wobei die Verarbeitungseinrichtung zusätzlich den ersten Innendruck verarbeitet, um die Zellkonzentration der Dosierflüssigkeit zu ermitteln. Hierdurch kann der Einfluss eines Füllstands der in dem ersten Dosierflüssigkeitsreservoir aufgenommenen Dosierflüssigkeit auf die Ermittlung der Zellkonzentration der Dosierflüssigkeit reduziert werden. Ein höherer Füllstand in dem ersten Dosierflüssigkeitsreservoir führt unabhängig von einer Beaufschlagung des ersten Dosierflüssigkeitsreservoirs mit Druckfluid zu einem höheren ersten Innendruck, wodurch wiederum ein höherer Fluiddruck resultiert. Dieser Einfluss führt bei der Ermittlung der Zellkonzentration der Dosierflüssigkeit zu einer gewissen Ungenauigkeit, die jedoch dadurch reduziert werden kann, dass der erste Innendruck berücksichtigt wird. In ähnlicher Weise kann alternativ oder in Ergänzung ein dem ersten Dosierflüssigkeitsreservoir zugeordneter erster Füllstandssensor vorgesehen sein.

Bevorzugt weist die Dosiervorrichtung ferner einen dem zweiten Dosierflüssigkeitsreservoir zugeordneten zweiten Reservoirdrucksensor auf. Ferner bevorzugt wird während der Ermittlung des Fluiddruckverlaufs mit dem zweiten Reservoirdrucksensor ein in dem zweiten Dosierflüssigkeitsreservoir ausgebildeter zweiter Innendruck ermittelt, wobei die Verarbeitungseinrichtung zusätzlich den ersten Innendruck verarbeitet, um die Zellkonzentration der Dosierflüssigkeit zu ermitteln. Alternativ oder in Ergänzung ist ein dem zweiten Dosierflüssigkeitsreservoir zugeordneter zweiter Füllstandssensor vorgesehen.

Eine erfindungsgemäße Dosiervorrichtung weist einen Fluidkanal, ein Ventil, eine Druckquelle, ein erstes Dosierflüssigkeitsreservoir, in dem eine Dosierflüssigkeit aufnehmbar ist, eine Verarbeitungseinrichtung, und einen dem Fluidkanal zugeordneten Fluidkanaldrucksensor auf, wobei der Fluidkanal einen ersten Fluidkanalabschnitt und einen fluidisch mit dem ersten Fluidkanalabschnitt verbundenen zweiten Fluidkanalabschnitt aufweist, wobei das Ventil ein Ventilglied aufweist, das zwischen dem ersten und dem zweiten Fluidkanalabschnitt angeordnet ist, und das in eine Offenstellung, in der die fluidische Verbindung des ersten Fluidkanalabschnitts mit dem zweiten Fluidkanalabschnitt freigegeben ist, und in eine Schließstellung bewegt werden kann, in der die fluidische Verbindung des ersten Fluidkanalabschnitts mit dem zweiten Fluidkanalabschnitt geschlossen ist, wobei das erste Dosierflüssigkeitsreservoir fluidisch mit dem ersten Fluidkanalabschnitt verbunden ist, wobei die Dosierflüssigkeit biologische Zellen aufweist, wobei die Verarbeitungseinrichtung dazu eingerichtet ist, ein vorstehend beschriebenes Verfahren auszuführen. Das vorstehend im Zusammenhang mit dem erfindungsgemäßen Verfahren zu den einzelnen Komponenten der Dosiervorrichtung Genannte gilt in gleicher Weise für die Komponenten der erfindungsgemäßen Dosiervorrichtung.

Vorzugsweise ist das Ventil als Schlauchquetschventil ausgebildet. Das Schlauchquetschventil umgreift den Fluidkanal, der insbesondere zumindest im betreffenden Bereich als flexibler Schlauch ausgebildet ist. Um das Ventilglied in die Schließstellung zu bewegen, wird das Ventilglied derart von außen auf den Fluidkanal gepresst, dass lokal ein Innendurchmesser des Fluidkanals so weit reduziert wird, dass ein Transport von Dosierflüssigkeit im betreffenden Bereich verhindert wird. Der Fluidkanal wird dementsprechend lokal zusammengequetscht. Damit kann verhindert werden, dass das Ventil, insbesondere das Ventilglied, mit der Dosierflüssigkeit in Berührung kommt. Derart kann das erfindungsgemäße Verfahren steril ausgeführt werden, was beispielsweise bei der Verwendung von Dosierflüssigkeiten, die bei der Herstellung von Zellkulturen für therapeutische Zwecke (CAR-T Zellproduktion) oder anderen präparativen Zellkulturmethoden vorteilhaft ist. Ferner vorzugsweise ist die Druckquelle als Schlauchquetschpumpe ausgebildet.

Alternativ oder in Ergänzung ist das Ventil als mediengetrenntes Membranventil ausgebildet. Ferner alternativ kann das Ventil als Kolbenschieberventil oder als Sitzventil ausgebildet sein.

Vorzugsweise ist das Ventil magnetisch wirkend ausgebildet. Das heißt, dass das Ventil als Magnetventil ausgebildet ist. Derart wird die Bewegung des Ventilgliedes durch Bestromen eines Elektromagneten bewirkt. Durch die Bewegung des Ventilglieds kann ein zwischen dem ersten Fluidkanalabschnitt und dem zweiten Fluidkanalabschnitt ausgebildeter Ventilsitz wahlweise freigegeben oder blockiert werden.

Bevorzugt weist die Dosiervorrichtung einen Temperatursensor auf, mit dem eine Temperatur der Dosierflüssigkeit ermittelbar ist. Ferner bevorzugt wird bei der Ermittlung der Zellkonzentration der Dosierflüssigkeit die Temperatur der Dosierflüssigkeit berücksichtigt. Die Aktivität beziehungsweise Bewegung beziehungsweise Beweglichkeit einiger biologischer Zellen wird durch deren Temperatur beziehungsweise die Temperatur der Dosierflüssigkeit, in der die biologischen Zellen aufgenommen sind, beeinflusst. In einem gewissen Temperaturbereich steigt die Aktivität der biologischen Zellen mit der Temperatur. Somit liegt bei hoher Temperatur eine hohe Aktivität der biologischen Zellen vor und bei geringer Temperatur eine geringe Aktivität der biologischen Zellen. Die Aktivität der biologischen Zellen wiederum kann die Viskosität der Dosierflüssigkeit und damit unter Berücksichtigung der vorstehend genannten Wirkzusammenhänge den Fluiddruckverlauf beeinflussen. Hierdurch kann eine gewisse Ungenauigkeit bei der Ermittlung der Zellkonzentration der Dosierflüssigkeit resultieren, die wiederum dadurch reduziert werden kann, dass die Temperatur der Dosierflüssigkeit bei der Ermittlung der Zellkonzentration der Dosierflüssigkeit berücksichtigt wird.

Ein erfindungsgemäßer Bioreaktor umfasst eine vorstehend beschriebene Dosiervorrichtung. Das vorstehend im Zusammenhang mit dem erfindungsgemäßen Verfahren zu dem Bioreaktor Genannte gilt in gleicher Weise für den erfindungsgemäßen Bioreaktor.

Nachfolgend wird die Erfindung anhand der beiliegenden Zeichnung näher erläutert und in dieser zeigen
- Fig. 1: eine Dosiervorrichtung mit einem Ventil, bei dem sich ein Ventilglied in einer Offenstellung befindet,
- Fig. 2: die in Fig. 1 dargestellte Dosiervorrichtung, wobei sich das Ventilglied in der Schließstellung befindet,
- Fig. 3: eine weitere Dosiervorrichtung mit einer Dosierspitze,
- Fig. 4: eine weitere Dosiervorrichtung,
- Fig. 5: die in Fig. 3 dargestellte Dosierspitze in einer Detailansicht,
- Fig. 6: ein Verfahren zum Ermitteln einer Zellkonzentration einer Dosierflüssigkeit,
- Fig. 7: eine Fluiddruckkurve eines schwach beeinflussten Eingangsdruckes,
- Fig. 8: eine Fluiddruckkurve eines stark beeinflussten Eingangsdruckes,
- Fig. 9: eine Zellkonzentrationskurve,
- Fig. 10: eine Dosierflüssigkeit mit einer hohen Zellkonzentration, und
- Fig. 11: eine Dosierflüssigkeit mit einer geringen Zellkonzentration.

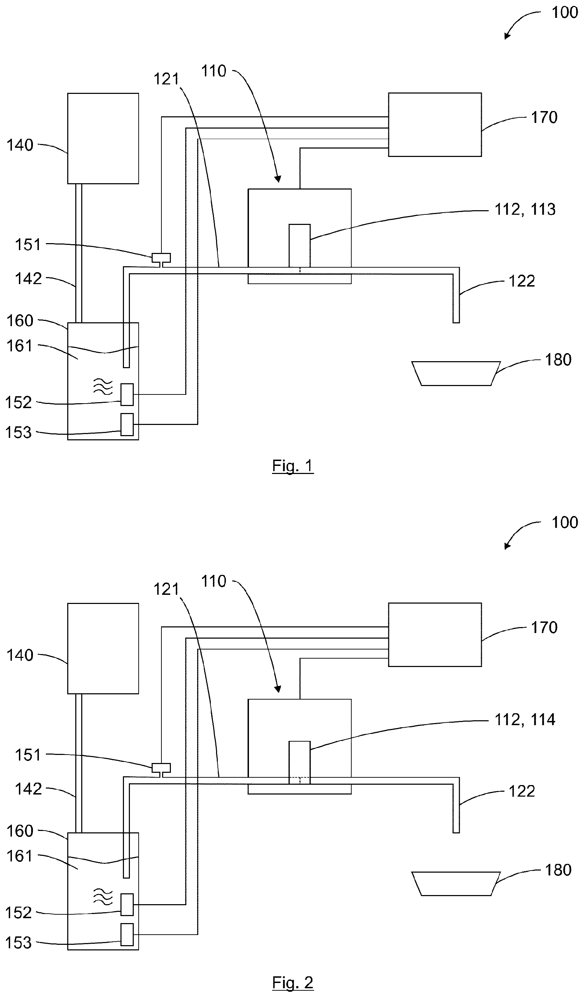

Fig. 1 zeigt eine Dosiervorrichtung 100. Die Dosiervorrichtung 100 weist ein Ventil 110 mit einem Ventilglied 112 auf. Die Dosiervorrichtung 100 weist einen Fluidkanal auf, der einen ersten Fluidkanalabschnitt 121 und einen zweiten Fluidkanalabschnitt 122 aufweist. Der erste Fluidkanalabschnitt 121 ist fluidisch mit dem zweiten Fluidkanalabschnitt 122 verbunden.

Die Dosiervorrichtung 100 weist zudem ein Ventil 110 mit einem Ventilglied 112 auf. Das Ventilglied 112 ist zwischen dem ersten Fluidkanalabschnitt 121 und dem zweiten Fluidkanalabschnitt 122 angeordnet. Das Ventilglied 112 ist in eine Offenstellung 113 bewegbar, in der die fluidische Verbindung des ersten Fluidkanalabschnitts 121 mit dem zweiten Fluidkanalabschnitt 122 freigegeben ist. Bei der in Fig. 1 dargestellten Dosiervorrichtung 100 befindet sich das Ventilglied 112 in der Offenstellung 113. Das Ventilglied 112 kann zudem von der Offenstellung 113 in eine Schließstellung 114 bewegt werden, in der die fluidische Verbindung des ersten Fluidkanalabschnitts 121 mit dem zweiten Fluidkanalabschnitt 122 geschlossen ist. Bei der in Fig. 2 dargestellten Dosiervorrichtung 100 befindet sich das Ventilglied 112 in der Schließstellung 114.

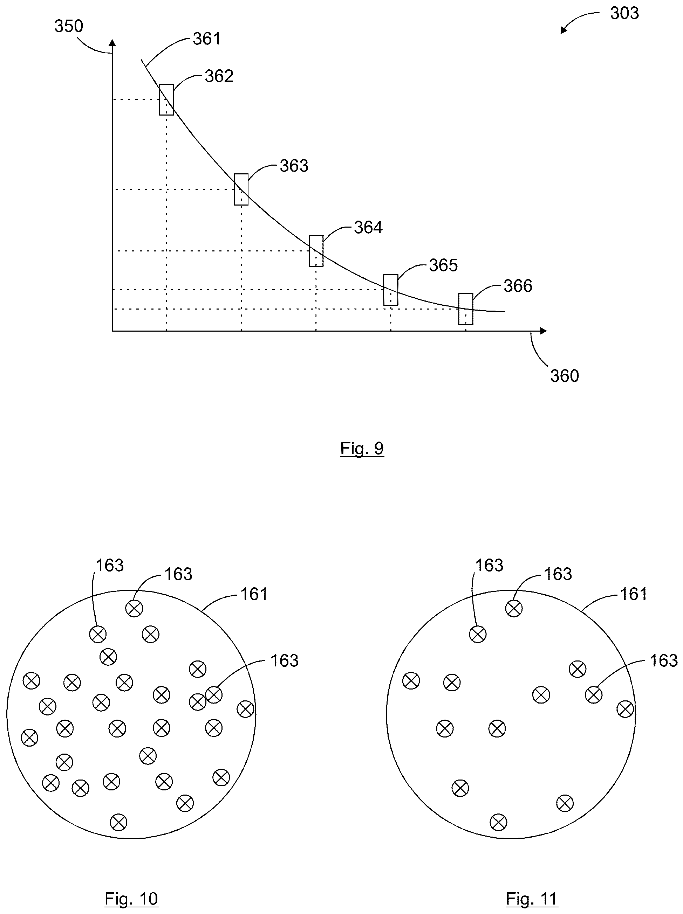

Die Dosiervorrichtung 100 weist ein erstes Dosierflüssigkeitsreservoir 160 auf, in dem eine Dosierflüssigkeit 161 aufgenommen ist. Die Dosierflüssigkeit 161 weist biologische Zellen 163 auf (vgl. Fig. 10 und 11). Rein exemplarisch ist das erste Dosierflüssigkeitsreservoir 160 fluidisch mit dem ersten Fluidkanalabschnitt 121 verbunden, indem der erste Fluidkanalabschnitt 121 in die Dosierflüssigkeit 161 eintaucht.

Die Dosiervorrichtung 100 weist eine Druckquelle 140 auf, mittels der das erste Dosierflüssigkeitsreservoir 160 mit einem Druckfluid beaufschlagbar ist. Hierzu ist die Druckquelle 140 mittels einem Druckfluidkanal 142 mit dem ersten Dosierflüssigkeitsreservoir 160 verbunden.

Die Dosiervorrichtung 100 weist einen dem Fluidkanal zugeordneten Fluidkanaldrucksensor 151 auf. Rein exemplarisch ist der Fluidkanaldrucksensor 151 dem ersten Fluidkanalabschnitt 121 zugeordnet, sodass der Fluidkanaldrucksensor 151 ausgehend von dem ersten Dosierflüssigkeitsreservoir 160 vor dem Ventil 110 angeordnet ist.

Rein exemplarisch weist die Dosiervorrichtung 100 einen Nährstoffsensor 152 auf, mit dem eine Nährstoffkonzentration der Dosierflüssigkeit 161 ermittelbar ist. Bevorzugt ist der Nährstoffsensor 152 in dem ersten Dosierflüssigkeitsreservoir 160 angeordnet. Ferner bevorzugt ist der Nährstoffsensor 152 in die Dosierflüssigkeit 161 eingetaucht.

Ferner exemplarisch weist die Dosiervorrichtung 100 einen dem ersten Dosierflüssigkeitsreservoir 160 zugeordneten ersten Reservoirdrucksensor 153 auf, mit dem ein in dem ersten Dosierflüssigkeitsreservoir 160 ausgebildeter erster Innendruck ermittelbar ist. Bevorzugt ist der erste Reservoirdrucksensor 153 in dem ersten Dosierflüssigkeitsreservoir 160 angeordnet. Ferner bevorzugt ist der erste Reservoirdrucksensor 153 in die Dosierflüssigkeit 161 eingetaucht.

Die Dosiervorrichtung 100 weist eine Verarbeitungseinrichtung 170 auf, die jeweils elektrisch mit dem Fluidkanaldrucksensor 151, dem Nährstoffsensor 152, dem ersten Reservoirdrucksensor 153 und dem Ventil 110 verbunden ist.

Rein exemplarisch ist hinter beziehungsweise unter dem dem ersten Fluidkanalabschnitt 121 abgewandten Ende des zweiten Fluidkanalabschnittes 122 ein Probenbehälter 180 angeordnet, in den die aus dem zweiten Fluidkanalabschnitt 122 ausströmende Dosierflüssigkeit 161 aufgenommen werden kann.

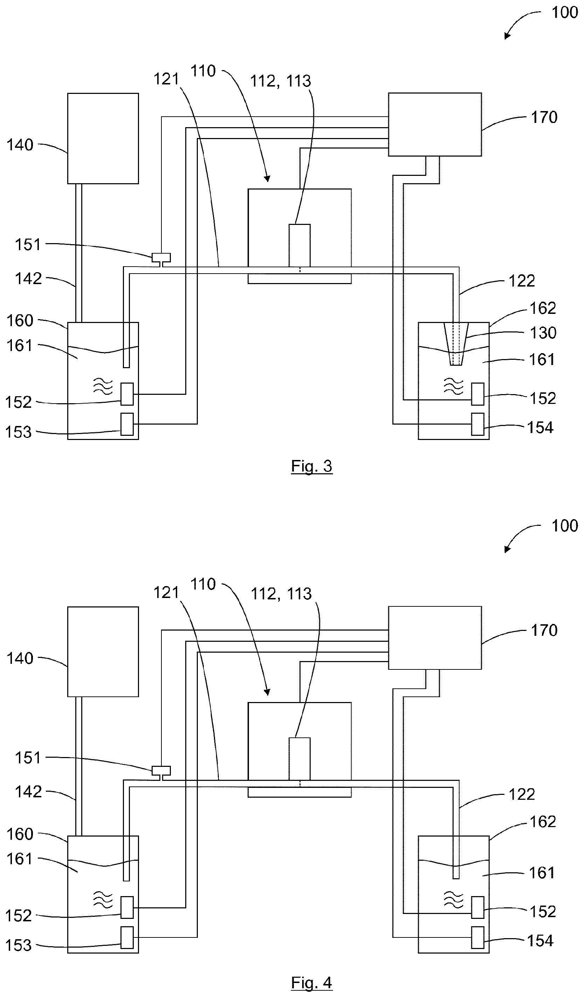

Die Fig. 3 und 4 zeigen jeweils eine weitere Dosiervorrichtung 100. Die in den Fig. 3 und 4 dargestellte jeweilige Dosiervorrichtung 100 weist jeweils ein zweites Dosierflüssigkeitsreservoir 162 auf. Der zweite Fluidkanalabschnitt 122 mündet in das zweite Dosierflüssigkeitsreservoir 162 ein.

Rein exemplarisch weist die Dosiervorrichtung 100 einen weiteren Nährstoffsensor 152 auf, der in dem zweiten Dosierflüssigkeitsreservoir 162 angeordnet ist. Auch mit dem weiteren Nährstoffsensor 152 ist eine Nährstoffkonzentration der Dosierflüssigkeit 161 ermittelbar. Wie auch der dem ersten Dosierflüssigkeitsreservoir 160 zugeordnete Nährstoffsensor 152 ist der dem zweiten Dosierflüssigkeitsreservoir 162 zugeordnete weitere Nährstoffsensor 152 bevorzugt in die Dosierflüssigkeit 161 eingetaucht.

Ferner exemplarisch weist die Dosiervorrichtung 100 einen dem zweiten Dosierflüssigkeitsreservoir 162 zugeordneten zweiten Reservoirdrucksensor 154 auf, mit dem ein in dem zweiten Dosierflüssigkeitsreservoir 162 ausgebildeter zweiter Innendruck ermittelbar ist. Bevorzugt ist der zweite Reservoirdrucksensor 154 in dem zweiten Dosierflüssigkeitsreservoir 162 angeordnet. Ferner bevorzugt ist der zweite Reservoirdrucksensor 154 in die Dosierflüssigkeit 161 eingetaucht.

Bei der in Fig. 3 dargestellten Dosiervorrichtung 100 weist der zweite Fluidkanalabschnitt 122 eine Dosierspitze 130 auf. Die Dosierspitze 130 ist an dem dem ersten Fluidkanalabschnitt 121 abgewandten Ende des zweiten Fluidkanalabschnittes 122 angeordnet. Die Dosierspitze 130 wirkt als Drossel. Rein exemplarisch taucht die Dosierspitze 130 in die in dem zweiten Dosierflüssigkeitsreservoir 162 aufgenommene Dosierflüssigkeit 161 ein.

Bei der in Fig. 4 dargestellten Dosiervorrichtung 100 taucht rein exemplarisch das dem ersten Fluidkanalabschnitt 121 abgewandte Ende des zweiten Fluidkanalabschnittes 122 in die in dem zweiten Dosierflüssigkeitsreservoir 162 aufgenommene Dosierflüssigkeit 161 ein.

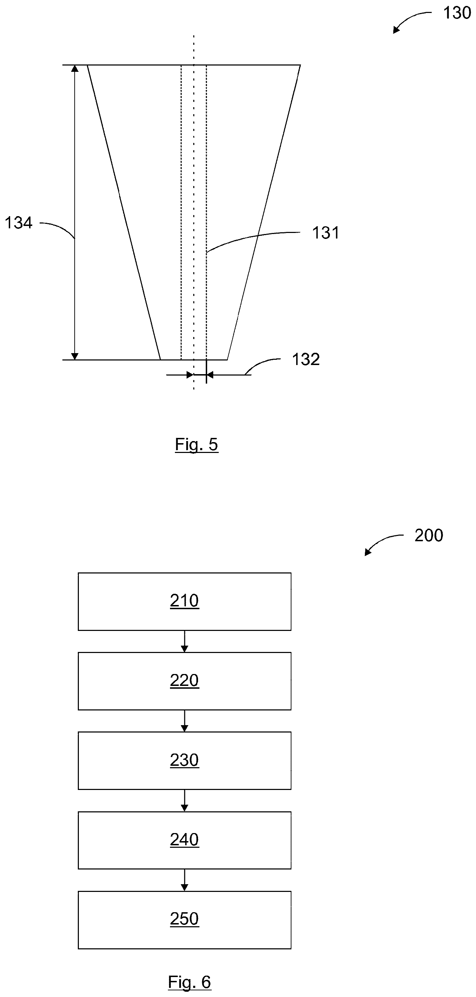

Fig. 5 zeigt die Dosierspitze 130 der in Fig. 3 dargestellten Dosiervorrichtung 100. Die Dosierspitze 130 weist eine Innengeometrie 131 auf, die mit der Dosierflüssigkeit 161 in Kontakt steht. Rein exemplarisch ist die Innengeometrie 131 kreiszylindrisch ausgebildet und weist eine Länge 134 und einen Radius 132 auf. Rein exemplarisch ist die Außengeometrie der Dosierspitze 130 konisch ausgebildet. Darüber hinaus sind andersartige Au-ßengeometrien der Dosierspitze 130 möglich, beispielsweise kann die Außengeometrie der Dosierspitze 130 kreiszylindrisch ausgebildet sein.

Die Verarbeitungseinrichtung 170 ist dazu eingerichtet, das in Fig. 6 dargestellte Verfahren 200 auszuführen. Das Verfahren 200 wird unter Verwendung der vorstehend beschriebenen Dosiervorrichtung 100 durchgeführt. Insbesondere wird hierbei davon ausgegangen, dass im Vorfeld eine Entlüftung des Fluidkanals vorgenommen wurde, so dass der gesamte Fluidkanal, also der erste Fluidkanalabschnitt 121 und der zweite Fluidkanalabschnitt 122, mit der Dosierflüssigkeit 161 gefüllt ist.

Das Verfahren 200 beginnt mit einem ersten Schritt 210, bei dem das erste Dosierflüssigkeitsreservoir 160 mit einem Druckfluid mittels der Druckquelle 140 beaufschlagt wird, sodass die Dosierflüssigkeit 161 durch Druckbeaufschlagung in dem ersten Fluidkanalabschnitt 121 bewegt wird.

Es folgt ein zweiter Schritt 220, bei dem das Ventilglied 112 zu Beginn einer Dosierzeit von der Schließstellung 114 in die Offenstellung 113 bewegt wird, sodass die Dosierflüssigkeit 161 in den zweiten Fluidkanalabschnitt 122 bewegt wird und aus dem zweiten Fluidkanalabschnitt 122 ausströmt.

Bei einem darauffolgenden dritten Schritt 230 wird das Ventilglied 112 zum Ende der Dosierzeit von der Offenstellung 113 in die Schließstellung 114 bewegt.

Es folgt ein vierter Schritt 240, bei dem ein Fluidruckverlauf während der Dosierzeit und/oder nach der Dosierzeit mit dem Fluidkanaldrucksensor 151 ermittelt wird.

Schließlich folgt ein fünfter Schritt 250, bei dem die Verarbeitungseinrichtung 170 den Fluiddruckverlauf verarbeitet, um eine Zellkonzentration der Dosierflüssigkeit 161 zu ermitteln.

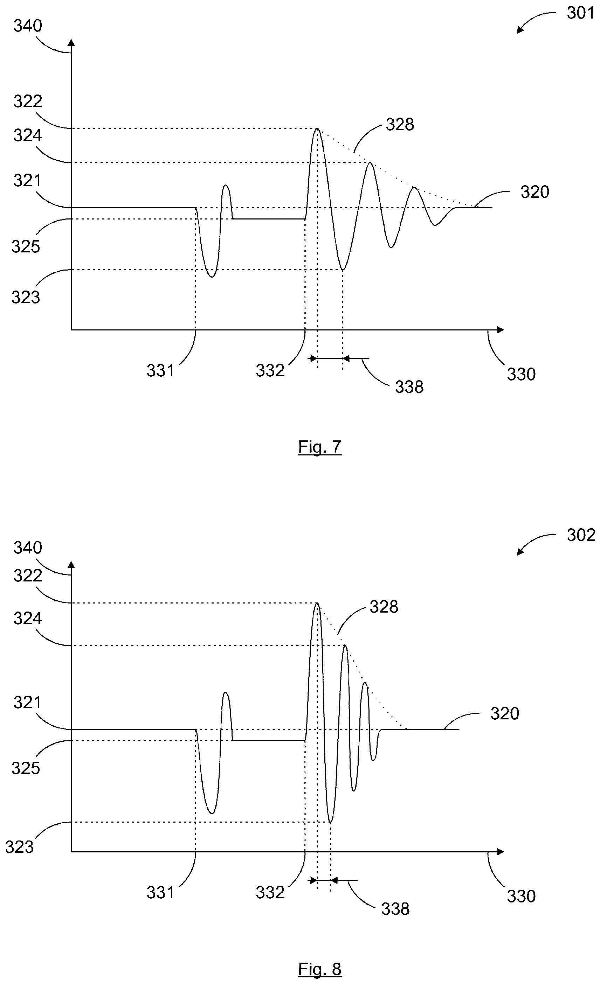

Fig. 7 zeigt ein Diagramm 301 mit einer Fluiddruckkurve 320 eines schwach beeinflussten Fluiddrucks und Fig. 8 ein Diagramm 302 mit einer Fluiddruckkurve 320 eines stark beeinflussten Fluiddrucks. Unter der Annahme ansonsten gleicher Bedingungen, insbesondere einer gleichen Dosiervorrichtung 100 und eines gleichen von der Druckquelle 140 bereitgestellten Druckes, entspricht die in Fig. 7 dargestellte Fluiddruckkurve 320 einer Dosierflüssigkeit 161, die eine höhere Zellkonzentration aufweist als die Dosierflüssigkeit 161, der der in Fig. 8 dargestellte Fluiddruckkurve 320 zugeordnet ist. Mit anderen Worten wird der in Fig. 7 mit der Fluiddruckkurve 320 dargestellte Fluiddruckverlauf stärker gedämpft als der in Fig. 8 mit der Fluiddruckkurve 320 dargestellte Fluiddruckverlauf.

Die Fluiddruckkurve 320 entspricht einem auf einer Druckachse 340 über einer Zeitachse 330 aufgetragenen zeitlichen Verlauf eines Fluiddrucks. Der Fluiddruck wird mit dem Fluidkanaldrucksensor 151 bestimmt (vgl. Fig. 1 bis 4). Zu Beginn entspricht der Fluiddruck einem ersten Fluiddruck 321. Zu einem Zeitpunkt zu Beginn des Dosierens 331 resultiert eine Druckschwingung, die sich darin äußert, dass der Fluiddruck abrupt abfällt und sich danach bei einem fünften Fluiddruck 325 einpendelt. Der fünfte Fluiddruck 325 liegt etwas unterhalb des ersten Fluiddrucks 321. Die Differenz zwischen dem ersten Fluiddruck 321 und dem fünften Fluiddruck 325 ist dadurch bedingt, dass in der Offenstellung 113, das heißt zwischen dem Zeitpunkt zu Beginn des Dosierens 331 und einem Zeitpunkt zum Ende des Dosierens 332, über den Fluidkanal und insbesondere über die Dosierspitze 130 ein Druckverlust auftritt.

Nach dem Zeitpunkt zum Ende des Dosierens 332 resultiert eine Druckschwingung, die sich darin äußert, dass der Fluiddruck abrupt ansteigt, bis als Extremwert mit einem zweiten Fluiddruck 322 ein Maximum erreicht wird. Danach fällt der Fluiddruck ab, bis als Extremwert mit einem dritten Fluiddruck 323 ein Minimum erreicht wird. Danach steigt der Fluiddruck wieder, bis als Extremwert mit einem vierten Fluiddruck 324 ein Maximum erreicht wird. Schließlich pendelt sich der Fluiddruck wieder bei dem ersten Fluiddruck 321 ein, wobei hierbei einige weitere jeweils als Maximum oder als Minimum ausgebildete Extremwerte erreicht werden, die der Übersichtlichkeit halber in den Fig. 7 und 8 nicht mit Bezugszeichen versehen sind.

In den Fig. 7 und 8 ist zudem jeweils eine Zeitdifferenz 338 zwischen dem Zeitpunkt, zu dem der Fluiddruck den zweiten Fluiddruck 322 erreicht, und dem Zeitpunkt, zu dem der Fluiddruck den dritten Fluiddruck 323 erreicht, abgebildet. Diese Zeitdifferenz 338 kann abhängig von der dynamischen Viskosität der Dosierflüssigkeit 161 sein und damit von deren Zellkonzentration. Rein exemplarisch ist die in Fig. 7 dargestellte Zeitdifferenz 338, die einer Dosierflüssigkeit 161 mit einer hohen Zellkonzentration zugeordnet ist, größer als die in Fig. 8 dargestellte Zeitdifferenz 338, die einer Dosierflüssigkeit 161 mit einer geringen Zellkonzentration zugeordnet ist. Die Dosierzeit entspricht der Differenz zwischen der Zeit zum Zeitpunkt zum Ende des Dosierens 332 und der Zeit zum Zeitpunkt zu Beginn des Dosierens 331. Die Zeitdifferenz 338 kann neben den Druckextremwerten 322, 323, 324 als weiterer Indikator für die dynamische Viskosität der Dosierflüssigkeit 161 verwendet werden. Gleichwohl ist es möglich, dass bei Dosierflüssigkeiten 161 mit unterschiedlicher dynamischer Viskosität ein Unterschied zwischen den jeweiligen Druckextremwerten 322, 323, 324 feststellbar ist, so wie in den Fig. 7 und 8 dargestellt, jedoch kein Unterschied in der Zeitdifferenz 338, das heißt also im Unterschied zu den in den Fig. 7 und 8 dargestellten Fluiddruckkurven 320.

Die Abweichungen der Amplitude des Eingangsdrucks zwischen den Druckextremwerten 322, 323, 324 und dem ersten Fluiddruck 321 ist bei der in Fig. 7 dargestellten Fluiddruckkurve 320, der eine Dosierflüssigkeit 161 mit einer geringen Zellkonzentration zugeordnet ist, geringer als bei der in Fig. 8 dargestellten Fluiddruckkurve 320, der eine Dosierflüssigkeit 161 mit einer hohen Zellkonzentration zugeordnet ist. Zudem ist eine Abklingrate 328 der Amplituden von aufeinanderfolgenden Druckextremwerten 322, 324 bei der in Fig. 8 dargestellten Fluiddruckkurve 320 größer als die Abklingrate 328 der in Fig. 7 dargestellten Fluiddruckkurve 320.

Fig. 9 zeigt ein Diagramm 303 mit einer Zellkonzentrationskurve 361. Die Zellkonzentrationskurve 361 entspricht einem auf einer Extremwertachse 350 über einer Zellkonzentrationsachse 360 aufgetragenen Verlauf einer Zellkonzentration einer Dosierflüssigkeit 161 in Abhängigkeit von einem Extremwert, der anhand des der Dosierflüssigkeit 161 zugeordneten Fluiddruckverlaufes bestimmt wird (vgl. Fig. 7 und 8). Als Extremwert wird insbesondere eine Differenz zwischen dem dritten Fluiddruck 323 und dem ersten Fluiddruck 321 verwendet. Diese Differenz wird auch als Amplitude des dritten Extremwertes bezeichnet. Der erste Fluiddruck 321 fungiert dabei als Normaldruck, nämlich als der Fluiddruck, der sich bei konstant geschlossenem Ventil 110 ausbildet. Mit zunehmender Zellkonzentration, das heißt entlang der Zellkonzentrationsachse 360 mit zunehmenden Werten sinkt der auf der Extremwertachse 350 aufgetragene Extremwert. Beispielhaft sinkt die Differenz zwischen dem dritten Fluiddruck 323 und dem ersten Fluiddruck 321 mit zunehmender Zellkonzentration. Dies ist auch anhand der in den Fig. 7 und 8 dargestellten Fluiddruckkurven 320 nachzuvollziehen.

Rein exemplarisch sind in dem in Fig. 9 dargestellten Diagramm 303 fünf Zellkonzentrationsklassen 362, 363, 364, 365, 366 dargestellt, nämlich eine erste Zellkonzentrationsklasse 362, eine zweite Zellkonzentrationsklasse 363, eine dritte Zellkonzentrationsklasse 364, eine vierte Zellkonzentrationsklasse 365 und eine fünfte Zellkonzentrationsklasse 366. Jede Zellkonzentrationsklasse 362, 363, 364, 365, 366 umfasst einen gewissen Extremwertbereich, was sich anhand der Erstreckung der betreffenden Zellkonzentrationsklasse 362, 363, 364, 365, 366 entlang der Extremwertachse 350 äußert und einen gewissen Zellkonzentrationsbereich, was sich anhand der Erstreckung der betreffenden Zellkonzentrationsklasse 362, 363, 364, 365, 366 entlang der Zellkonzentrationsachse 360 äußert. Bevorzugt sind in Bezug auf zumindest eine der Achsen Extremwertachse 350 und Zellkonzentrationsachse 360 benachbarte Zellkonzentrationsklassen 362, 363, 364, 365, 366 derart beabstandet, dass eine eindeutige Zuordnung zu einer der Zellkonzentrationsklassen 362, 363, 364, 365, 366 ermöglicht wird. In diesem Zusammenhang bedeutet eindeutige Zuordnung, dass verhindert wird, dass ein aus einem Extremwert und einer Zellkonzentration bestehendes Wertepaar mehreren Zellkonzentrationsklassen 362, 363, 364, 365, 366 zugeordnet werden kann, das heißt, dass sich an der betreffenden Stelle mehrere Zellkonzentrationsklassen 362, 363, 364, 365, 366 überschneiden.

Fig. 10 zeigt eine Dosierflüssigkeit 161 mit einer hohen Zellkonzentration und Fig. 11 eine Dosierflüssigkeit 161 mit einer geringen Zellkonzentration. Wie bereits vorstehend erwähnt weist die Dosierflüssigkeit 161 biologische Zellen 163 auf. In den Fig. 10 und 11 sind aus Gründen der Übersichtlichkeit jeweils lediglich drei biologische Zellen 163 mit einem Bezugszeichen versehen. Die in Fig. 10 dargestellte Dosierflüssigkeit 161 weist mehr biologischen Zellen 163 auf als die in Fig. 11 dargestellte Dosierflüssigkeit, was damit einhergeht, dass die in Fig. 10 dargestellte Dosierflüssigkeit 161 eine höhere Zellkonzentration aufweist als die in Fig. 11 dargestellte Dosierflüssigkeit 161. Beispielsweise kann die in Fig. 10 dargestellte Dosierflüssigkeit 161 der in Fig. 7 dargestellten Fluiddruckkurve 320 zugeordnet sein und die in Fig. 11 dargestellte Dosierflüssigkeit 161 der in Fig. 8 dargestellten Fluiddruckkurve 320.

## Patentansprüche

1. Verfahren zum Ermitteln einer Zellkonzentration einer Dosierflüssigkeit unter Verwendung einer Dosiervorrichtung (100) mit einem Fluidkanal, einem Ventil (110), einer Druckquelle (140), einem ersten Dosierflüssigkeitsreservoir (160), in dem eine Dosierflüssigkeit aufgenommen ist, einer Verarbeitungseinrichtung (170), und einem dem Fluidkanal zugeordneten Fluidkanaldrucksensor (151), wobei der Fluidkanal einen ersten Fluidkanalabschnitt (121) und einen fluidisch mit dem ersten Fluidkanalabschnitt (121) verbundenen zweiten Fluidkanalabschnitt (122) aufweist, wobei das Ventil (110) ein Ventilglied (112) aufweist, das zwischen dem ersten Fluidkanalabschnitt (121) und dem zweiten Fluidkanalabschnitt (122) angeordnet ist, und das in eine Offenstellung (113), in der die fluidische Verbindung des ersten Fluidkanalabschnitts (121) mit dem zweiten Fluidkanalabschnitt (122) freigegeben ist, und in eine Schließstellung (114) bewegt werden kann, in der die fluidische Verbindung des ersten Fluidkanalabschnitts (121) mit dem zweiten Fluidkanalabschnitt (122) geschlossen ist, wobei das erste Dosierflüssigkeitsreservoir (160) fluidisch mit dem ersten Fluidkanalabschnitt (121) verbunden ist, wobei die Dosierflüssigkeit biologische Zellen aufweist, mit den Schritten: Beaufschlagen des ersten Dosierflüssigkeitsreservoirs (160) mit einem Druckfluid mittels der Druckquelle (140), sodass die Dosierflüssigkeit durch Druckbeaufschlagung in dem ersten Fluidkanalabschnitt (121) bewegt wird, Bewegen des Ventilgliedes (112) zu Beginn einer Dosierzeit von der Schließstellung (114) in die Offenstellung (113), sodass die Dosierflüssigkeit in den zweiten Fluidkanalabschnitt (122) bewegt wird und aus dem zweiten Fluidkanalabschnitt (122) ausströmt, Bewegen des Ventilgliedes (112) zum Ende der Dosierzeit von der Offenstellung (113) in die Schließstellung (114), Ermitteln eines Fluidruckverlaufs während der Dosierzeit und/oder nach der Dosierzeit mit dem Fluidkanaldrucksensor (151), wobei die Verarbeitungseinrichtung (170) den Fluiddruckverlauf verarbeitet, um eine Zellkonzentration der Dosierflüssigkeit zu ermitteln.

2. Verfahren nach Anspruch 1, wobei der zweite Fluidkanalabschnitt (122) eine Dosierspitze (130) aufweist, wobei die Dosierspitze (130) an einem Ende des zweiten Fluidkanalabschnittes (122) angeordnet ist, das dem ersten Fluidkanalabschnitt (121) abgewandt ist, wobei die Dosierflüssigkeit in Folge dessen, dass die Dosierflüssigkeit aus dem zweiten Fluidkanalabschnitt (122) ausströmt, aus der Dosierspitze (130) ausströmt,

3. Verfahren nach Anspruch 2, wobei die Verarbeitungseinrichtung (170) zusätzlich einen Geometriekennwert der Dosierspitze (130) verarbeitet, um die Zellkonzentration der Dosierflüssigkeit zu ermitteln.

4. Verfahren nach einem der vorhergehenden Ansprüche, wobei unmittelbar nachdem das Ventilglied (112) von der Schließstellung (114) in die Offenstellung (113) bewegt wurde oder unmittelbar nachdem das Ventilglied (112) von der Offenstellung (113) in die Schließstellung (114) bewegt wurde, mit dem Fluidkanaldrucksensor (151) ein Fluiddruckschwingungsverlauf bestimmt wird, wobei auf Basis des Fluiddruckschwingungsverlaufs die Zellkonzentration der Dosierflüssigkeit ermittelt wird.

5. Verfahren nach Anspruch 4, wobei die Zellkonzentration der Dosierflüssigkeit mit zumindest einem Kennwert aus der Gruppe: Amplitude des ersten Extremwerts, Amplitude des zweiten Extremwerts, Amplitude des dritten Extremwerts, Zeitdifferenz zwischen dem ersten Extremwert und/oder dem zweiten Extremwert und/oder dem dritten Extremwert, Abklingrate der Amplitude zumindest zweier aufeinanderfolgender Extremwerte, ermittelt wird, der anhand des Fluiddruckschwingungsverlaufs bestimmt wird.

6. Verfahren nach einem der vorhergehenden Ansprüche, wobei vor der Ermittlung der Zellkonzentration der Dosierflüssigkeit die Verarbeitungseinrichtung (170) parametriert wird, indem für zumindest eine Testflüssigkeit mit bekannter Zellkonzentration die folgenden Schritte durchgeführt werden: Bewegen der Testflüssigkeit in dem Fluidkanal mittels Bewegen des Ventilgliedes (112) von der Schließstellung (114) zu der Offenstellung (113) und anschließend Bewegen des Ventilgliedes (112) von der Offenstellung (113) zu der Schließstellung (114), wobei in Zusammenhang mit dem Bewegen der Testflüssigkeit in dem Fluidkanal unmittelbar nachdem das Ventilglied (112) von der Schließstellung (114) in die Offenstellung (113) bewegt wurde oder unmittelbar nachdem das Ventilglied (112) von der Offenstellung (113) in die Schließstellung (114) bewegt wurde, mit dem Fluidkanaldrucksensor (151) ein Test-Fluiddruckschwingungsverlauf bestimmt wird, wobei auf Basis des Test-Fluiddruckschwingungsverlaufs die Zellkonzentration der Testflüssigkeit ermittelt wird, wobei die Zellkonzentration der Testflüssigkeit mit zumindest einem Kennwert aus der Gruppe: Amplitude des ersten Extremwerts, Amplitude des zweiten Extremwerts, Amplitude des dritten Extremwerts, Zeitdifferenz zwischen dem ersten Extremwert und/oder dem zweiten Extremwert und/oder dem dritten Extremwert, Abklingrate der Amplitude zumindest zweier aufeinanderfolgender Extremwerte, ermittelt wird, der anhand des Test-Fluiddruckschwingungsverlaufs bestimmt wird, wobei anschließend zumindest eine Zellkonzentrationsklasse unter Berücksichtigung der ermittelten Zellkonzentration der zumindest einen Testflüssigkeit ermittelt wird, wobei bei der anschließenden Ermittlung der Zellkonzentration bei entsprechender Übereinstimmung der Dosierflüssigkeit die zumindest eine Zellkonzentrationsklasse zugeordnet wird, wobei mit dieser zugeordneten zumindest einen Zellkonzentrationsklasse die Zellkonzentration der Dosierflüssigkeit ermittelt wird.

7. Verfahren nach Anspruch 6, wobei die Verarbeitungseinrichtung (170) mit mehreren Testflüssigkeiten mit jeweils bekannten, unterschiedlichen Zellkonzentrationen parametriert wird, wobei mehrere Zellkonzentrationsklassen unter Berücksichtigung der jeweils für die mehreren Testflüssigkeiten ermittelten Zellkonzentration ermittelt werden.

8. Verfahren nach Anspruch 6 oder 7, **dadurch gekennzeichnet, dass** die unterschiedlichen Viskositätsklassen unter Verwendung eines zellkonzentrationsbezogenen Modells für maschinelles Lernen ermittelt werden.

9. Verfahren nach einem der vorhergehenden Ansprüche, wobei die Dosiervorrichtung (100) ein zweites Dosierflüssigkeitsreservoir (162) aufweist, wobei das zweite Dosierflüssigkeitsreservoir (162) fluidisch mit dem zweiten Fluidkanalabschnitt (122) verbunden ist, wobei die aus dem zweiten Fluidkanalabschnitt (122) ausströmende Dosierflüssigkeit in das zweite Dosierflüssigkeitsreservoir (162) einströmt.

10. Verfahren nach Anspruch 9, wobei zwischen einem in dem ersten Dosierflüssigkeitsreservoir (160) ausgebildeten ersten Innendruck und einem in dem zweiten Dosierflüssigkeitsreservoir (162) ausgebildeten zweiten Innendruck ein Unterschied von zumindest 60 mbar besteht.

11. Verfahren nach einem der vorhergehenden Ansprüche, wobei die Dosiervorrichtung (100) Teil eines Bioreaktors ist, wobei die Dosierflüssigkeit ferner einen Nährstoff aufweist.

12. Verfahren nach Anspruch 11, wobei die Dosiervorrichtung (100)ferner einen Nährstoffsensor (152) aufweist, wobei mit dem Nährstoffsensor (152) eine Nährstoffkonzentration der Dosierflüssigkeit ermittelt wird, wobei die Verarbeitungseinrichtung (170) zusätzlich die Nährstoffkonzentration der Dosierflüssigkeit verarbeitet, um die Zellkonzentration der Dosierflüssigkeit zu ermitteln.

13. Verfahren nach einem der vorhergehenden Ansprüche, wobei der Fluidkanaldrucksensor (151) derart angeordnet ist, dass der Fluidkanaldrucksensor (151) die Probenflüssigkeit nicht unmittelbar berührt, wobei die Druckquelle (140) als hydraulische Druckquelle (140) ausgebildet ist und als Druckfluid eine Hydraulikflüssigkeit verwendet wird, wobei das erste Dosierflüssigkeitsreservoir (160) von außerhalb mit dem Druckfluid mittels der Druckquelle (140) beaufschlagt wird.

14. Verfahren nach einem der vorhergehenden Ansprüche, wobei die Dosiervorrichtung (100) ferner einen dem ersten Dosierflüssigkeitsreservoir (160) zugeordneten ersten Reservoirdrucksensor (153) aufweist, wobei während der Ermittlung des Fluiddruckverlaufs mit dem ersten Reservoirdrucksensor (153) ein in dem ersten Dosierflüssigkeitsreservoir (160) ausgebildeter erster Innendruck ermittelt wird, wobei die Verarbeitungseinrichtung (170) zusätzlich den ersten Innendruck verarbeitet, um die Zellkonzentration der Dosierflüssigkeit zu ermitteln.

15. Dosiervorrichtung mit einem Fluidkanal, einem Ventil (110), einer Druckquelle (140), einem ersten Dosierflüssigkeitsreservoir (160), in dem eine Dosierflüssigkeit aufnehmbar ist, einer Verarbeitungseinrichtung (170), und einem dem Fluidkanal zugeordneten Fluidkanaldrucksensor (151), wobei der Fluidkanal einen ersten Fluidkanalabschnitt (121) und einen fluidisch mit dem ersten Fluidkanalabschnitt (121) verbundenen zweiten Fluidkanalabschnitt (122) aufweist, wobei das Ventil (110) ein Ventilglied (112) aufweist, das zwischen dem ersten und dem zweiten Fluidkanalabschnitt (122) angeordnet ist, und das in eine Offenstellung (113), in der die fluidische Verbindung des ersten Fluidkanalabschnitt (121) mit dem zweiten Fluidkanalabschnitt (122) freigegeben ist, und in eine Schließstellung (114) bewegt werden kann, in der die fluidische Verbindung des ersten Fluidkanalabschnitt (121) mit dem zweiten Fluidkanalabschnitt (122) geschlossen ist, wobei das erste Dosierflüssigkeitsreservoir (160) fluidisch mit dem ersten Fluidkanalabschnitt (121) verbunden ist, wobei die Dosierflüssigkeit biologische Zellen aufweist, wobei die Verarbeitungseinrichtung (170) dazu eingerichtet ist, ein Verfahren nach einem der vorhergehenden Ansprüche auszuführen, wobei bevorzugt das Ventil (110) als Schlauchquetschventil ausgebildet ist und/oder wobei bevorzugt das Ventil (110) magnetisch wirkend ausgebildet ist.
